# EUROPEAN PATENT APPLICATION

(11) **EP 4 215 106 A1**
(43) Date of publication of application: **26.07.2023**
(21) Application number: 23153193.0
(22) Date of filing: 25.01.2023
(51) Int. Cl.: A61B 5/00, A41H 1/00, A61B 5/107, G01B 11/25, G06T 7/11

(54) **APPARATUS FOR THE AUTOMATIC DETECTION OF ANTHROPOMETRIC MEASUREMENTS AND FOR THE DETERMINATION OF SIZES OF ITEMS OF CLOTHING ON THE BASIS OF SAID MEASUREMENT, AND CORRESPONDING METHOD**

(30) Priority: 25.01.2022 IT 202200001205
(71) Applicant: Jaes Informatica Societa'Cooperativa, 42122 Reggio Emilia (RE) (IT)
(72) Inventor: Campani, Samuel, 42122 Reggio Emilia (RE) (IT)
(74) Representative: Petraz, Davide Luigi

(57) **Abstract**

The invention concerns a processing apparatus (10) for the automatic detection of anthropometric measurements and the determination of sizes of items of clothing, comprising a stereoscopic image acquisition device (11), a processing unit (12) for processing said images which is configured for the extraction of a skeleton (103) of the person (100) from at least one stereoscopic image (50) and to process said skeleton (103) and said stereoscopic image (50) in order to obtain one or more anthropometric measurements of the person (100), and a control unit (18). The invention also concerns a processing method for the automatic measurement of anthropometric measurements and the determination of sizes of items of clothing.

## Description

### FIELD OF THE INVENTION

The present invention concerns a processing apparatus for the automatic detection of anthropometric measurements with the purpose of determining the correct sizes of items of clothing for different individuals, and a corresponding method.

The processing apparatus and method can be used, by way of a non-restrictive example, in sectors such as the supply of work clothes, in particular for large companies with many employees, in clothing stores, in tailors' shops, but also in autonomous stations positioned in points of interest such as shopping centers, shopping streets or suchlike. Furthermore, they are also suitable for use by users who wish to buy clothing online on the various marketplaces.

### BACKGROUND OF THE INVENTION

Choosing and purchasing an item of clothing is known to be difficult, due to the need to test various items before identifying the one that best suits the person's body. In addition to taking time, it is an unhygienic operation since the garments tested come into contact with various parts of the person's body.

For example, in large workplaces where thousands of workers work wearing uniforms, such as factories or hospitals, there is the rather complex problem of matching each worker with a uniform of the correct size.

Another problem is to identify the correct size when the purchase has to be made online: the item of clothing has to be sent to the home of the person who, if it is not the right size, must return it to the sender with considerable logistical costs that erode the seller's profit margins.

To date, a known solution is the manual measurement of the person, for example of each worker, by a tailor, but this entails an increase in costs and long and laborious procedures. Alternatively, it is known that the employer purchases or rents uniforms, by statistically selecting the different quantities of available sizes, but this is often inefficient, with the consequence of having a high number of returns or the need to make additional orders.

Devices are known which provide to detect the size starting from one or more images of the person, also from different angles.

The reliability of the above known solutions is proportional to the number of images available which, as they increase, make the method complex, long and laborious, and significantly correlated to the person's skill in creating the images.

Some known solutions provide a revolving platform on which the person is positioned. However, this solution can entail high risks of malfunctioning, in particular if subjected to intensive use, and also requires frequent and costly maintenance interventions.

Alternative solutions provide devices, such as laser scanners or suchlike, which rotate around the person, immobile in a central position. However, these solutions are expensive, and also require bulky installations due to the mechanisms used to move the devices.

Other solutions provide that the person wears a special test uniform adherent to the body, for example made of Lycra, on which markers are positioned by means of which the three-dimensional features of the person are reconstructed, and the necessary measurements calculated. The entire process to recognize the size is not easily applied, also because the selection of the uniform implies, in a certain sense, an a priori estimate of the size. These solutions also require a large number of images to be acquired, and in any case need to include the times and costs of shipping the test uniform. Finally, these solutions are not very precise, since the measurements can include errors even up to +/- 10 cm.

The patent document US 2014/0300907 A1 describes a system for determining and adapting items of clothing, accessories or prostheses, comprising a sensor to detect the energy reflected by a person and a module to measure portions of the person's body.

Document IT 2019 0002 1252 A1 describes a system and a method to estimate sufficiently accurate body measurements of a user for the production of bespoke clothing. The system includes a mobile processing device, such as a smartphone or tablet, into which the user enters personal information relating to his/her body, such as height, weight, age and standard size.

The patent document US 2019/0357615 A1 describes systems and methods to extract measurements of a body using a mobile device.

Another disadvantage of these known solutions is that none is completely automated, since the user must always identify him/herself, providing one or more identifying data and possibly personal information relating to his/her body, and send the information entered and the measurements acquired to whomsoever is involved.

There is therefore a need to perfect an apparatus for the automatic detection of anthropometric measurements and for determining the sizes of items of clothing, and a corresponding method, which can overcome at least one of the disadvantages of the state of the art.

In particular, one purpose of the present invention is to provide an apparatus and to perfect a method for detecting the size of a person autonomously, and therefore without necessarily requiring the intervention of an operator. The automation of the method can make it possible to reduce times and costs, at the same time guaranteeing people's privacy since the method does not require sensitive personal data.

Another purpose is to ensure that the person does not have to enter personal information, such as his/her identifying data.

Another purpose is to provide an apparatus able to autonomously manage the anthropometric measurements and sizes determined, memorizing them internally or sending them to remote management and storage systems.

Another purpose is to ensure that the person does not have to perform repetitive, time-consuming and laborious operations to acquire the images.

Another purpose of the present invention is to provide an apparatus which is not too bulky, so that it can be easily positioned even in narrow places such as a dressing room of a shop or the changing rooms of a workplace.

Another purpose is to provide a low-cost device, so that it can also be easily purchased by companies with a limited number of employees or by freelance workers such as tailors, who can thus speed up their work.

The Applicant has devised, tested and embodied the present invention to overcome the shortcomings of the state of the art and to obtain these and other purposes and advantages.

### SUMMARY OF THE INVENTION

The present invention is set forth and characterized in the independent claims. The dependent claims describe other characteristics of the present invention or variants to the main inventive idea.

In accordance with the above purposes, and to resolve the technical problem disclosed above in a new and original way, also achieving considerable advantages compared to the state of the prior art, a processing apparatus for the automatic detection of anthropometric measurements and the determination of sizes of items of clothing comprises an image acquisition device, a processing unit for processing the images and a control unit.

The image acquisition device is a stereoscopic device. The processing unit is configured for the extraction of a skeleton of the person from at least one stereoscopic image and to process the skeleton and the stereoscopic image in order to obtain one or more anthropometric measurements of the person. The control unit is configured to query databases in which standard anthropometric measurements which are associated with the different sizes of items of clothing are stored, and to determine a size of the person by comparing the one or more anthropometric measurements of the person with the standard anthropometric measurements.

In accordance with one aspect of the present invention, the processing apparatus comprises an identification codes reader configured to automatically detect identifying parameters of a person.

Doing so achieves at least the advantage that the intervention of operators or the involvement of the person is not required in the operations of acquiring the anthropometric measurements and of determining the size, since the apparatus can operate autonomously. Thanks to the present invention, the apparatus is completely automated, also allowing to associate the name of the person being measured first with the images, and then with his/her size.

Another advantage is the possibility of achieving very high precision in determining the measurements of the person required to determine their size, with errors even lower than 5 mm, which guarantee minimum errors in the production of the item of clothing.

An additional advantage is that the acquisition of stereoscopic images requires only a stereoscopic acquisition device comprising two optical sensors, positioned fixed and in a known relative position with respect to each other, and does not require moving parts that can wear/break over time, unlike for example laser scanners which need to be moved in order to perform the scans.

Moreover, the use of stereoscopic images does not require the person to perform a long and repetitive series of rotations around a longitudinal axis thereof in order to achieve the required precision in the measurements.

Advantageously, the apparatus does not have excessive overall sizes and can be manufactured at low cost.

Some embodiments of the present invention also concern a processing method for the automatic detection of anthropometric measurements and the determination of sizes of items of clothing comprising a step of detecting, in an automatic manner, the identifying parameters of a person by means of an identification codes reader. It also comprises a step of acquiring at least one stereoscopic image defining the outline of a person by means of an image acquisition device, a step of extracting a skeleton of the person from the at least one stereoscopic image, a step of processing the skeleton and the stereoscopic image, in order to obtain one or more anthropometric measurements of the person, a step of querying a database in which standard anthropometric measurements which are associated with the different sizes of items of clothing are stored, a step of determining a size of the person by comparing the one or more anthropometric measurements of the person with the standard anthropometric measurements stored in the database.

### DESCRIPTION OF THE DRAWINGS

These and other aspects, characteristics and advantages of the present invention will become apparent from the following description of some embodiments, given as a non-restrictive example with reference to the attached drawings wherein:
- fig. 1 is a three-dimensional representation of a processing apparatus for the automatic detection of anthropometric measurements and for the determination of sizes of items of clothing according to the present invention;
- fig. 2a is a three-dimensional image acquired by the apparatus of fig. 1;
- fig. 2b is the image of a schematized skeleton obtained from the three-dimensional image of fig. 2a;
- fig. 3a is a three-dimensional representation of a person extracted from the three-dimensional image of fig. 2a;
- fig. 3b is a cross section of the three-dimensional representation of fig. 3a, according to the plane IIIb-IIIb;
- fig. 4 is a schematic representation of a user interface on an interface device comprised in the apparatus of fig. 1;
- fig. 5 is a representation of some steps of the processing method for the automatic detection of anthropometric measurements and for the determination of sizes of items of clothing according to the present invention;
- figs. 6a, 6b are three-dimensional images of an object acquired by the apparatus of fig. 1.

We must clarify that in the present description the phraseology and terminology used, as well as the figures in the attached drawings also as described, have the sole function of better illustrating and explaining the present invention, their function being to provide a non-limiting example of the invention itself, since the scope of protection is defined by the claims.

To facilitate comprehension, the same reference numbers have been used, where possible, to identify identical common elements in the drawings. It is understood that elements and characteristics of one embodiment can be conveniently combined or incorporated into other embodiments without further clarifications.

### DESCRIPTION OF SOME EMBODIMENTS OF THE PRESENT INVENTION

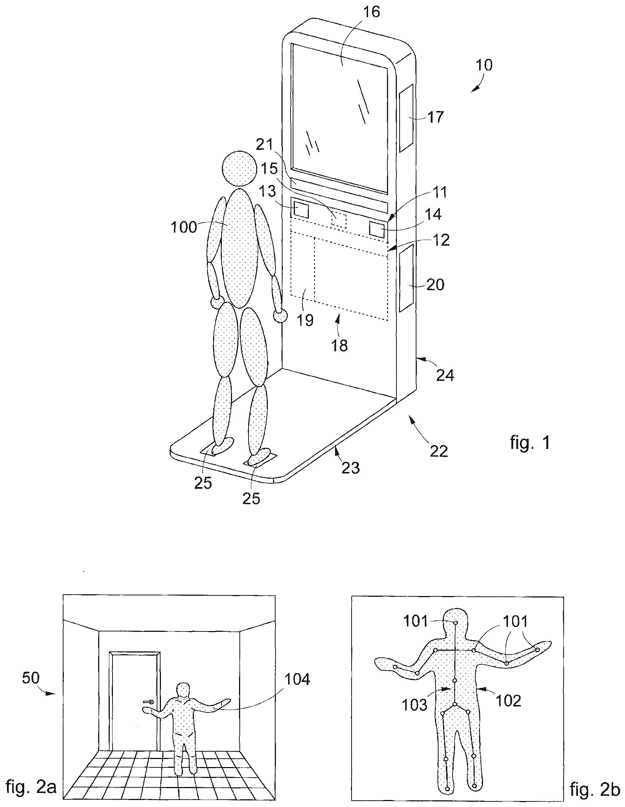

With reference to fig. 1, an apparatus 10 for the automatic detection of anthropometric measurements and the determination of sizes of items of clothing comprises a stereoscopic image acquisition device 11, a processing unit 12 for processing the images, a control unit 18 and an identification codes reader 20 configured to automatically detect identifying parameters of a person 100.

The identification codes reader 20 can be a barcode reader, a QR code reader, an RFID (Radiofrequency Identifier) reader, an NFC (Near Field Communication) reader or suchlike.

The identification codes reader 20 can for example be associated with a personal badge. By the term associate, we mean that the identification codes reader 20 is able to read the badge's information, for example by framing a barcode, a QR code, or by reading an RFID or NFC tag, or suchlike.

Advantageously, it can therefore automatically detect the identifying parameters of the person 100. In this way, it is possible to automatically associate the anthropometric measurements and/or the size measured with each person 100.

The apparatus 10 can comprise a support structure 22.

According to one embodiment, the support structure 22 can comprise a base 23 and a substantially vertical element 24.

The base can provide indicators 25 for positioning the person 100 in front of the stereoscopic image acquisition device 11, such as signs traced on the base, one or more light signals projected by a suitable device (not shown in the drawings) of the apparatus 10 or suchlike.

The element 24 can house the image acquisition device 11, and possibly both the processing unit 12 and also the control unit 18, as well as other components of the apparatus 10 described in greater detail below.

According to one variant, not shown in the drawings, the support structure can be configured as a cabin, for example a changing room cabin, in which the person 100 can be accommodated and in which the image acquisition device 11 is housed.

According to another variant, not shown in the drawings, the apparatus 10 can be integrated into a personal device of the person 100, such as for example a smartphone, a personal computer, a tablet, or suchlike. For example, this solution can be advantageously applied for online purchases on online stores. In this case, the support structure is the personal device's case; the image acquisition device 11 comprises or cooperates with the camera(s) of the personal device and the image analysis algorithm is implemented in the CPU of the personal device.

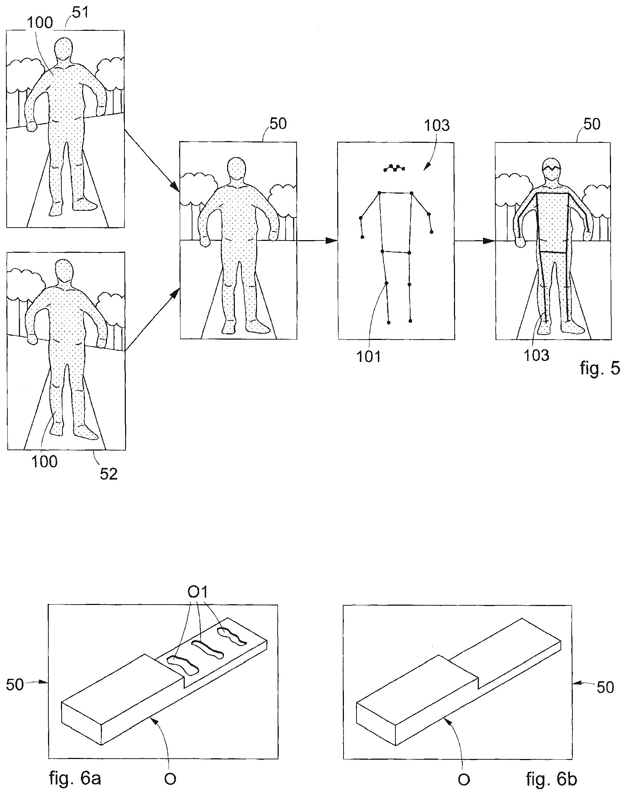

The image acquisition device 11 can be any stereoscopic camera, telecamera or video camera whatsoever, comprising two optical sensors 13, 14 and an extraction element 15 for extracting a stereoscopic image 50 (fig. 2a, 5, 6a, 6b) starting from a right 51 and left 52 image (fig. 5) which have been acquired by the respective optical sensors 13, 14.

The extraction element 15, and the processing unit 12 and control unit 18, can each comprise a processing module, or CPU, and a data storage module. For example, the CPU can be any form of computer processor whatsoever usable in computing. The storage module can be connected to the CPU and can consist of one or more commercially available memories, such as a random access memory (RAM), a read-only memory (ROM), a hard disk, a mass memory, a volatile memory or any other form of digital storage whatsoever. They can also comprise auxiliary circuits, for example at least one of either cache circuits, power supply circuits, clock circuits, subsystems and suchlike, and can be connected to the CPU in a conventional manner.

According to some embodiments, the processing unit 12 can be integrated into the image acquisition device 11 and comprise or cooperate with the element 15 for extracting a stereoscopic image 50. For example, the functions of the extraction element 15 and of the processing unit 12 can be performed by a single element inside the image acquisition device 11.

According to some embodiments, the control unit 18 can comprise or cooperate with the processing unit 12. For example, the functions of the control unit 18 and of the processing unit 12 can be performed by a single device. The control unit 18 and/or the processing unit 12 can comprise input/output (I/O) circuits, comprising communication devices of the wired or wireless type, such as for example devices with RJ45, Bluetooth, WIFI technology and/or suchlike. They can allow communication toward external systems, such as a network of supervisors which comprises other devices, a communication network, a server, a database or similar, and suchlike.

The control unit 18 and/or the processing unit 12 can be configured to send the anthropometric measurements and/or the size of the person 100 to external management and storage systems. In particular, the sending can occur in automatic mode.

The external management and storage systems can, for example, be order management systems of a clothing manufacturer or suchlike, systems that communicate with machines or plants for manufacturing items of clothing or suchlike.

Preferably, the image acquisition device 11 comprises wide type lenses, which offer greater scene coverage. It can also comprise an IMU (Inertial Measurement Unit) device to compensate for any inclinations during installation.

The stereoscopic image acquisition device 11 simulates human vision in order to estimate the depth of the scene located in front of the device.

The implementation of the stereo vision provides to use the images 51, 52 in addition to the knowledge of some parameters and characteristics of the optical sensors 13, 14, such as the distance between them for example.

The processing unit 12 is configured to perform an analysis of at least one stereoscopic image 50 detected by the image acquisition device 11 for the identification of a plurality of key points 101 in the outline 102 of a person 100 (fig. 2b).

In particular, the processing unit 12 is configured to extract the outline 102 of the person 100 starting from the stereoscopic image 50.

By key points 101 we mean points of the body of a person 100 useful for detecting the physique of the person 100, that is, useful for carrying out anthropometric measurements in accordance with the guidelines of the sector legislation, in particular of the ISO 8559-1 standard.

The key points 101 can be two or more of either the head, ears, eyes, nose, neck, shoulders, elbows, wrists, navel, pelvis, hips, knees, ankles, or suchlike. The key points 101 can be indexed by an identifying parameter, or "ID". Each key point 101 can be associated with a normalized confidence score between 0.0 and 1.0, indicating the level of confidence in the identification of the key point 101 itself.

The analysis of the outline 102 alone is not in fact sufficient to carry out a correct, complete and reliable detection of the anthropometric measurements.

The anthropometric measurements can be chest/breast circumference, waist circumference, hip circumference, height, shoulder width, jacket length, sleeve length, trousers length, possibly arm circumference, thigh circumference and suchlike.

The processing unit 12 is also configured to organize the key points 101 into a virtual schematic "skeleton" 103, as represented in fig. 2b. In this example, the skeleton 103 was reconstructed using the following key points 101: wrists, elbows, shoulders, head, navel, pelvis, hips, knees, ankles.

The key points 101 organized into the skeleton 103 allow to identify the areas to be measured. For example, the skeleton 103 can be used to estimate the position of the person, so as to be able to determine where, within the image 50, an anatomical part of such person is located, such as the wrist, the elbow, the knee or suchlike. As an example, it is therefore possible to measure the length of the forearm based on the position of the wrist and elbow.

The analysis of the skeleton 103 also allows to better guide the person 100 in the method for acquiring the images 51, 52 described below, verifying that the person 100 has actually complied with the guidelines for correct acquisition.

The processing unit 12 is also configured to extract one or more of the anthropometric measurements as above from the skeleton 103 and from the stereoscopic image 50.

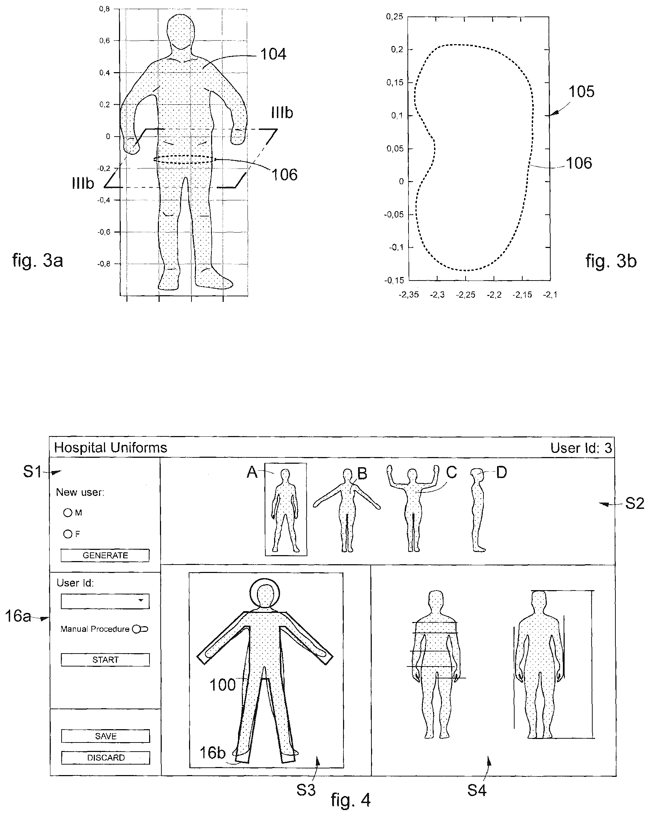

For the extraction of the one or more anthropometric measurements, the processing unit 12 can be configured to identify a plane, starting from the key points 101, and to create, for each measurement, a section 105 on a three-dimensional reconstruction 104 extracted from the stereoscopic image 50. For example and as shown in fig. 3a, it is possible to see the three-dimensional reconstruction 104 of the person 100 and the section 105 extracted at the level of the hip for the measurement. Having obtained the section 105, by approximating the points region, shown in fig. 3b, for example by means of an ellipse, the perimeter can be calculated. Finally, the size can be determined on the basis of one or more of the anthropometric measurements, as a function of specific data, for example organized in tables, databases or suchlike, supplied by the supplier of the items of clothing.

The processing unit 12 can comprise an algorithm for extracting anthropometric measurements of the person 100, stored in the storage module to command the CPU.

The extraction algorithm can be configured to perform the operations of analyzing at least one stereoscopic image 50, extracting the outline 102, organizing the key points 101 into a skeleton 103, extracting the one or more anthropometric measurements.

The extraction algorithm can be based on Deep Learning and/or artificial intelligence (AI) techniques, such as neural networks, fuzzy logic or suchlike. Advantageously, this allows to improve the analysis of the acquired images, allowing to reduce the errors in the determination of the key points 101, of the outline 102, of the skeleton 103 and in general in the determination of the anthropometric measurements and of the size.

In the storage module there can therefore be stored data relating to sets for training (Training sets) and validating (Validation sets) computer vision algorithms based on Deep Learning techniques and/or neural networks.

The processing unit 12 can be configured to perform at least one post-processing operation of the image 50 selected from a decimation operation, a spatial and/or temporal filtering operation and/or a filling operation.

Advantageously, the decimation operation can allow to reduce the computational load required for image processing, reducing the resolution of the processed data while maintaining the original quality as much as possible.

Advantageously, the application of the spatial and temporal filters allows to reduce the presence of artifacts in the image: as shown in fig. 6b, from the image 50 of an object O it is possible to reduce or cancel some roughness O1, present in the raw image 50 of fig. 6a, which does not belong to the morphology of the object O.

Again advantageously, the filling of holes in the images, often generated by homogeneous surfaces with no texture, can allow to have a more uniform reconstruction of the depth map.

The application of the operations as above can be fundamental in the case in question, because if the imperfections are minimal in a static scene and with geometric objects O such as the one represented in figs. 6a, 6b, in the case of small involuntary movements of a posing person, the reconstruction can be invalidated, decreasing the accuracy of the measurement, not to mention the greater complexity of the scene in topological terms.

The apparatus 10 can comprise one or more devices for interfacing with the person 100, selected from a monitor 16, audio devices 17, a keyboard, a mouse and/or suchlike. By way of example, the monitor 16 can be a touch screen. As another example, the audio devices 17 can comprise a microphone and/or a speaker.

The control unit 18 can comprise user interface circuits 19. The user interface circuits 19 can be configured to interface with the one or more interface devices as above.

The control unit 18 can be configured to control and manage the size acquisition operations.

In particular, the control unit 18 is configured to query databases in which standard anthropometric measurements, which are associated with the different sizes of items of clothing, are stored, and determine a size of the person 100 by comparing the one or more anthropometric measurements of the person 100 with the standard anthropometric measurements.

The control unit 18 can also be configured to perform one or more of the following operations:
- configuring the image acquisition device 11, setting its operating parameters such as the focus of the sensors 13, 14, the opening or closing of the lenses or suchlike;
- detecting identifying parameters of the person 100, such as name and surname, gender and/or identification number of a personal badge;
- explaining, by means of audio/images, how the method for acquiring the size will take place, in order to guide the person 100 in the acquisition method;
- allowing the user to view the data once the method has been completed;
- asking for confirmation before saving the data and proceeding with a new acquisition.

In particular, the control unit 18 can be configured to detect the identifying parameters of the person 100 by commanding the identification codes reader 20.

The detection of the identifying parameters of the person 100 allows to associate the anthropometric measurements and/or the size to the person 100.

Advantageously, thanks to the control unit 18, the apparatus 10 is able to determine, autonomously and therefore without requiring the intervention of an operator, the size of the person 100.

As shown by way of example in fig. 4, the method can provide to display a graphic interface 16a on the monitor 16 comprising a section S1 for the identifying parameters of the person 100, a section S2 with the pose or poses A, B, C, D, to be assumed, a section S3 in which the right 51 or left 52 image overlaps with an outline 16b representing one of the poses A, B, C, D, a section S4 with the calculated anthropometric measurements and/or similar sections.

The apparatus 10 can comprise an infrared (IR) projection device 21. The stereoscopic image acquisition device 11 can therefore be configured to detect images both in visible and also in infrared light.

The projection device 21 can project a static IR pattern, for example a cloud of dots, not visible to the naked eye, which can be detected by the image acquisition device 11. Advantageously, it is in this way possible to improve the accuracy of the depth detection, particularly in scenes with very uniform backgrounds, such as white walls.

The operation of the processing apparatus 10 for the automatic detection of anthropometric measurements and the determination of sizes of items of clothing described heretofore, which corresponds to the method according to the present invention, comprises the following steps:
- automatically detecting the identifying parameters of a person 100 by means of an identification codes reader 20;
- detecting at least one stereoscopic image 50 of the person 100;
- processing the at least one stereoscopic image 50 for the identification of a plurality of key points 101 in the outline 102 of the person 100 and the organization of the key points 101 into a schematized skeleton 103;
- extracting one or more anthropometric measurements of the person 100 from the skeleton 103 and from the stereoscopic image 50.

The method can comprise associating the identification codes reader 20 with a personal badge of the person 100. In particular and as shown in fig. 5, the method can provide to detect a right image 51 and a left image 52, by means of respective optical sensors 13, 14 of an image acquisition device 11.

The method can then provide to identify the points of correspondence between the two images 51, 52, and calculate their distance from the image acquisition device 11, thus creating a three-dimensional model, or stereoscopic image 50.

The organization of the key points 101 into a skeleton 103 can be done by joining the key points 101.

The method can provide to identify, from the skeleton 103 and from the stereoscopic image 50, an actual position assumed by the person 100 and the points in which to carry out the anthropometric measurements.

Advantageously, the method does not provide to recognize the person 100, but only to estimate, by identifying the key points 101, where the key joints of the body are located. In this way, a low computational load is maintained, which allows to use components and technologies with not excessive costs.

The method also comprises the steps of querying databases in which standard anthropometric measurements, which are associated with the different sizes of items of clothing, are stored, and of determining the size of the person 100 by comparing the one or more anthropometric measurements of the person 100 with the standard anthropometric measurements.

The method can provide to make the person 100 assume one or more predefined poses A, B, C, D. It can then provide to detect, for each pose A, B, C, D, at least two images of the person 100, respectively a right 51 and a left 52 image.

Advantageously, even one or in any case a limited number of poses A, B, C only in a position in front of the camera may be sufficient to obtain highly precise measurements, therefore without the need for the person to position him/herself at different angles with respect to the image acquisition device 11.

By way of example, it can provide the assumption of four poses A, B, C, D, represented in fig. 4.

Each pose A, B, C, D is designed to favor a certain type of anthropometric measurement, as disclosed below:
- pose A: facilitates the measurement of the legs and inner thighs;
- pose B: facilitates the measurement of the hips and waist zone;
- pose C: facilitates the measurement of the arms and bust/chest;
- pose D: can be used in a complementary manner to the others, in order to obtain more accurate measurement estimates, reducing blind spots due to perspective; only one of these poses is sufficient, not two on different sides.

The steps of the method as above, in particular the assumption of pose D, allow to avoid using rotating platforms which risk wearing out or breaking if subjected to intensive use.

The method can provide to guide the person 100 during the execution of the size acquisition method. For example, the person 100 can be guided in assuming the one or more poses A, B, C, D. For example, the indications can be given through audio communication, with vocal instructions, or shown on a monitor 16. In particular, in order to guarantee a better alignment, an outline indicative of the pose to be assumed can be shown on the monitor 16, such outline being made to overlap with the image of one of the optical sensors 13, 14.

The method can comprise a step of projecting a static IR pattern, for example a cloud of dots, not visible to the naked eye, which can be detected by the image acquisition device 11, in order to improve depth detection.

The method can provide that the apparatus 10 can supply at output only the anthropometric measurements and/or the size of the person 100, preferably only the size. In particular, it can provide to not save the images 50, 51, 52 and the three-dimensional reconstruction 104, but to process them in a volatile memory of the apparatus 10 (for example of the device 11, of the unit 12 or of the unit 18, preferably of the device 11), keeping them only for the time required to identify the anthropometric measurements and/or size. The method can also provide that the images 50, 51, 52 and the three-dimensional reconstruction 104 cannot be recovered once the size acquisition method has ended.

In particular, the method can provide to process the images 50, 51, 52 and the three-dimensional reconstruction 104 directly inside the image acquisition device 11 (edge processing) and to communicate to the control unit 18 only the extrapolated measurement data for filing according to law.

Advantageously, this allows to safeguard the person's privacy, since the images 50, 51, 52 and the three-dimensional reconstruction 104 can represent sensitive data, including images of people in scanty clothing or wearing only underwear.

The method can provide to maintain a pre-set distance between the device and the zone in which the person is positioned for the measurement, which can be comprised between 0.5 and 2 m, preferably between 0.75 and 1.5 m, even more preferably substantially 1 m.

The method can also provide to maintain a free space around the zone in which the person is positioned for the measurement, for example a free space with a radius from the center of the measurement zone of less than 1 m, preferably less than 0.5 m, even more preferably substantially 46 cm.

The method can provide to maintain lighting that is as controlled as possible, for example avoiding the interference of windows that could blind the system or create considerable shadows, precluding the success of the measurements.

In the variant suitable to be implemented on a personal device of the person 100, the method provides to carry out, before the steps described above, an initial set-up step in order to correctly parameterize the anthropometric measurements which will be detected subsequently. By way of example, it is provided that the person 100 prints a geometric shape, of known dimensions, such as a circle or a square, on a piece of paper. Subsequently, the person 100 will position him/herself in the chosen position to carry out the method according to the present invention and will take first images 51, 52 holding the sheet of paper in his/her hand. In this way, using algorithms of a known type, the measurements of the geometric shape will be detected and compared with the known dimensions in order to determine a conversion scale factor, to be applied to the measurements detected from the images 51, 52 of the person 100 which will be subsequently taken by means of the same personal device.

It is clear that modifications and/or additions of parts may be made to the apparatus 10 and to the method as described heretofore, without departing from the field and scope of the present invention, as defined by the claims.

It is also clear that, although the present invention has been described with reference to some specific examples, a person of skill in the art will be able to achieve other equivalent forms of processing method and apparatus for the automatic detection of anthropometric measurements and the determination of sizes of items of clothing, having the characteristics as set forth in the claims and hence all coming within the field of protection defined thereby.

In the following claims, the sole purpose of the references in brackets is to facilitate their reading and they must not be considered as restrictive factors with regard to the field of protection defined by the same claims.

## Claims

1. Processing apparatus (10) for the automatic detection of anthropometric measurements and the determination of sizes of items of clothing, comprising a stereoscopic image acquisition device (11), a processing unit (12) for processing said images which is configured for the extraction of a skeleton (103) of the person (100) from at least one stereoscopic image (50) and to process said skeleton (103) and said stereoscopic image (50) in order to obtain one or more anthropometric measurements of the person (100), and a control unit (18) configured to query databases in which standard anthropometric measurements, which are associated with the different sizes of items of clothing, are stored, thus determining a size of said person (100), **characterized in that** it comprises an identification codes reader (20) configured to automatically detect identifying parameters of said person (100).

2. Apparatus (10) as in claim 1, **characterized in that** said identification codes reader (20) is selected from a barcode reader, a QR code reader, an RFID (Radiofrequency Identifier) tag reader, an NFC (Near Field Communication) tag reader.

3. Apparatus (10) as in claim 1 or 2, **characterized in that** said identification codes reader (20) can be associated with a personal badge of said person (100).

4. Apparatus (10) as in any claim from 1 to 3, **characterized in that** it comprises an infrared projection device (21) and said stereoscopic image acquisition device (11) is configured to detect images both in visible and also in infrared light, **and in that** it comprises one or more devices for interfacing with the person (100) selected from a monitor (16), in particular a touch screen, and/or audio devices (17) configured to guide said person (100) during the execution of the method for acquiring sizes.

5. Apparatus (10) as in any claim from 1 to 4, **characterized in that** said processing unit (12) is configured to perform at least one of either an operation of decimation, spatial filtering and/or temporal filtering of the stereoscopic image (50).

6. Apparatus (10) as in any claim from 1 to 5, **characterized in that** said control unit (18) and/or processing unit (12) is configured to send said anthropometric measurements and said size of said person (100) to external management and storage systems **and in that** said sending occurs in automatic mode.

7. Processing method for the automatic detection of anthropometric measurements and the determination of sizes of items of clothing, **characterized in that** it comprises:
- a step of detecting, in an automatic manner, identifying parameters of a person (100) by means of an identification codes reader (20);
- a step acquiring, through an image acquisition device (11), at least one stereoscopic image (50) defining the outline (102) of the person (100);
- a step of extracting a skeleton (103) of the person (100) from said at least one stereoscopic image (50);
- a step of processing said skeleton (103) and said stereoscopic image (50) to obtain one or more anthropometric measurements of the person (100) which are compared with standard anthropometric measurements stored in a database;
- a step of determining a size of items of clothing which are suitable to be worn by the person (100).

8. Method as in claim 7, **characterized in that** it comprises associating said identification codes reader (20) with a personal badge of said person (100).

9. Method as in claim 7 or 8, **characterized in that** said person (100) assumes one or more predefined poses (A, B, C, D), which are suitable to optimize the acquisition of one or more measurements of the person (100) **and in that** at least two images are detected for each of said poses (A, B, C, D), respectively a right (51) and a left (52) image, by means of respective optical sensors (13, 14).

10. Method as in any claim from 7 to 9, **characterized in that** said extraction step comprises:
- a sub-step of identifying a plurality of key points (101) in said outline (102), selected from a group consisting of: ears, eyes, nose, neck, shoulders, elbows, wrists, hips, knees, ankles; and
- a sub-step of organizing said key points (101) to form said schematized skeleton (103);
**and in that** said anthropometric measurements of the person (100) are selected from a group consisting of chest/breast circumference, waist circumference, hips circumference, height, shoulder width, jacket length, sleeve length, trouser length, arm circumference and thigh circumference.

11. Method as in any claim from 7 to 10, **characterized in that** it comprises a step of projecting a static infrared pattern (IR) by means of a projection device (21), said infrared pattern (IR) being configured to be detected by said image acquisition device (11) in order to improve depth detection.

12. Method as in any claim from 7 to 11, **characterized in that** said one or more anthropometric measurements of the person (100) extracted in said processing step and said images (50, 51, 52) are stored in a volatile memory of said image acquisition device (11) **and in that,** in an output step, it is provided to supply as output to said person (100) only said size.

13. Method as in any claim from 7 to 12, **characterized in that** it comprises the execution of at least one of either a step of decimation, a step of spatial filtering and/or a step of temporal filtering of said stereoscopic image (50).

14. Method as in any claim from 7 to 13, **characterized in that** it comprises an initial set-up step in which an acquired image of a geometric shape of known sizes is compared with a stored image of said geometric shape in order to determine a conversion scale factor, to be applied to the anthropometric measurements thus detected.
